Europäisches Patentamt

European Patent Office

Office européen des brevets

(19)

(11) Numéro de publication: **0 059 159**
**B1**

(12) **FASCICULE DE BREVET EUROPEEN**

(45) Date de publication du fascicule du brevet:
**22.05.85**

(51) Int. Cl.⁴: **A 61 B 3/00,** B 23 K 26/02

(21) Numéro de dépôt: **82810044.6**

(22) Date de dépôt: **03.02.82**

(54) **Verre de contact pour l'observation ou l'irradiation de l'oeil.**

(30) Priorité: **12.02.81 CH 931/81**

(43) Date de publication de la demande:
**01.09.82 Bulletin 82/35**

(45) Mention de la délivrance du brevet:
**22.05.85 Bulletin 85/21**

(84) Etats contractants désignés:
**CH DE FR GB IT LI NL**

(56) Documents cités:
**EP - A - 0 030 210**
**FR - A - 2 248 814**
**FR - A - 2 384 478**
**GB - A - 2 049 216**
**US - A - 4 033 679**

(73) Titulaire: **LASAG AG, Bernstrasse 11, CH-3600 Thun (CH)**

(72) Inventeur: **Roussel, Philippe, Anemonenweg 1, NL-2241 HX Wassenaar (NL)**

(74) Mandataire: **Schneider, Jürg et al, c/o Société Générale de l'Horlogerie Suisse S.A. ASUAG Faubourg du Lac 6, CH-2501 Bienne (CH)**

ACTORUM AG

**Description**

La présente invention est relative à un verre de contact pour l'observation ou l'irradiation de l'œil.

Des verres de contact de type varié sont utilisés en ophtalmologie pour l'observation ou le traitement de l'œil par irradiation, notamment pour le traitement de la chambre antérieure de l'œil.

Les verres les plus souvent utilisés jusqu'à ce jour sont notamment le verre de contact de Koeppe et le verre de contact du type verre de Goldmann. L'utilisation de tels verres a été décrite en particulier dans la publication intitulée «Gonioskopie und Goniofotographie» de Winfried Müller et Hans-Peter Brandt, Ferdinand Enke Verlag Stuttgart 1979.

Le verre de contact de Koeppe est un verre qui présente une face d'entrée des faisceaux bombée et une face de sortie sensiblement sphérique destinée à être appliquée sur la zone transparente de la cornée. Ces verres toutefois n'ont jusqu'à ce jour été utilisés que pour l'observation de l'œil. Le dimensionnement de ces verres à été principalement étudié en vue d'obtenir une image de la chambre antérieure de l'œil avec un grandissement de 20 à 30 fois au cours des opérations d'observation.

Le verre de contact du type Goldmann comporte essentiellement une face d'intrée du rayonnement plane, une face de sortie sensiblement sphérique destinée à être appliquée sur la zone transparente de la cornée et au moins une face ou paroi réfléchissante permettant l'observation indirecte de la chambre antérieure de l'œil par réflexion totale des faisceaux sur cette paroi.

Malgré leurs qualités intrinsèques indéniables pour les opérations d'observation et, notamment, la maniabilité, la facilité et le confort d'utilisation pour le médecin ophtalmologue en ce qui concerne le verre de contact du type Goldmann, ces deux types de verres ne permettent pas le traitement de l'œil par irradiation avec toute garantie de sécurité pour le patient, de fiabilité et de reproductibilité du traitement notamment dans le cas d'irradiations à hautes énergies par rayon laser. En effet, dans ce type de traitement, un objectif essentiel est d'obtenir une densité d'énergie suffisante au voisinage du point de focalisation du rayonnement en vue de provoquer, par claquage optique du milieu diélectrique à l'intérieur de l'œil, une onde de pression susceptible d'assurer la perforation de parois de la chambre antérieure de l'œil avoisinantes. Cependant la sécurité du patient au cours de ces traitements exige une réduction maximale de la densité d'énergie rayonnée au niveau de la jonction cornée – verre de contact afin d'éviter tout dommage de celle-ci dans la zone d'entrée dans l'œil du faisceau laser. De plus, en vue d'assurer à chaque tir d'impulsion laser des conditions de reproductibilité et de stabilité d'émission de l'onde de pression (onde de choc) dans l'œil, il est indispensable que le verre de contact permette, par ses qualités de forme et de composition, la meilleure focalisation possible du faisceau laser utile et le maintien de la qualité de focalisation pour tout point de l'œil, principalement de la chambre antérieure de l'œil.

Les verres de contact de la technique antérieure ne permettent pas d'obtenir l'ensemble des conditions nécessaires à un traitement fiable, sûr et constant. En particulier on a pu constater que l'utilisation de verres de contact du type Goldmann tels que précédemment décrits ne permet pas dans certaines conditions d'utilisation, c'est-à-dire en définitive pour certains points de la chambre antérieure, d'obtenir une énergie suffisante au point de focalisation pour obtenir par claquage optique l'émission de l'onde de pression voulue. En fait, le phénomène de claquage optique pour ces points n'est susceptible d'être obtenue qu'au prix d'une augmentation substantielle de la densité d'énergie rayonnée du faisceau, cette augmentation d'un facteur 2 à 3 au moins étant prohibitive en raison des risques de dommage au niveau de la cornée et du verre de contact lui-même.

La présente invention permet de remédier aux inconvénients précités et a pour objet la mise en œuvre d'un verre de contact pour l'observation et le traitement de la chambre antérieure de l'œil.

Un autre objet de la présente invention est un verre de contact permettant, par irradiation laser de l'œil à travers ce verre, d'obtenir le phénomène de claquage optique et d'onde de pression du milieu interne de l'œil à partir d'énergie rayonnée par le faisceau la plus faible possible.

Un autre objet de la présente invention est un verre de contact permettant d'obtenir un phénomène de claquage optique stable et bien localisé spatialment, ce même verre de contact permettant d'obenir des effets sensiblement identiques, du point de vue clinique, pour des paramètres identiques de l'émission laser.

Un autre objet de la présente invention est un verre de contact pour lequel l'ensemble des caractéristiques précitées doit rester sensiblement invariant quelle que soit la position du point de tir dans l'œil, ou, pour un point de tir fixe, quelles que soient les positions relatives du faisceau laser et du verre de contact par rapport à l'œil.

Le verre de contact selon l'invention est défini par l'ensemble des caractéristiques techniques de l'actuelle revendication 1. De tels verres de contact peuvent être utilisés en chirurgie ophtalmologique notamment pour le diagnostic et le traitement d'affections telles que le glaucome ou la cataracte. Pour plus de détails à ce sujet on peut se reporter à la demande de brevet européen EP-A-0 030 210 (no 80 810 357.6) au nom de la demanderesse.

L'invention sera décrite en détail à l'aide de la description et des dessin ci-après dans lesquels les mêmes références représentent les mêmes éléments et où:

– la figure 1a représente, en perspective, un verre de contact selon l'invention,

– la figure 1b représente, en coupe selon un plan de symétrie longitudinal, le verre de contact de la figure 1a appliqueé contre la cornée d'un globe oculaire pour l'observation et le traitement de la chambre antérieure de l'œil,

– la figure 2 représente, également en coupe selon un plan de symétrie longitudinal, le verre de contact de la figure 1a appliqué contre la cornée d'un globe oculaire et l'image du faisceau utile sur la face d'entrée du verre de contact

– la figure 3 représente, à titre de résultat comparatif, les courbes d'aberration de focalisation d'un faisceau utile dans le cas de l'utilisation d'un verre de contact du type Goldmann et pour un verre de contact conformément à l'invention.

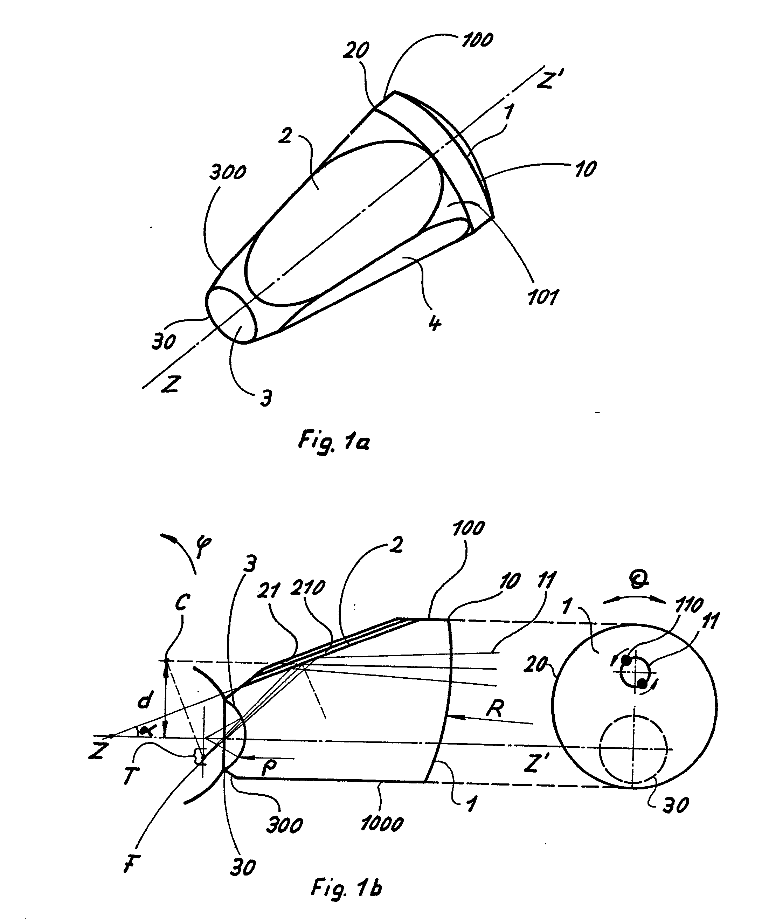

Conformément à la figure 1a, le verre de contact pour l'observation ou l'irradiation de la chambre antérieure de l'œil comprend pour le rayonnement utile une face d'entrée 1, une face réfléchissante 2 par réfléchissante 2 par réflexion totale de ce rayonnement et une surface de sortie 3 sensiblement sphérique. Le verre de contact tel que représenté figure 1 est appliqué directement sur la zone transparente de la cornée, la surface de sortie 3 sensiblement sphérique étant appliquée sur la cornée. Un produit de liaison du genre de ceux connus sous le nom commercial de «Methocel» permet d'assurer une bonne liaison entre le verre de contact et la cornée. Afin de diminuer au maximum les aberrations au point de focalisation du faisceau, la surface d'entrée 1 constitue pour le rayonnement utile une surface d'onde. Ainsi à l'interface air-verre de contact constitué par la surface d'entrée 1 le rayonnement utile ne subit aucune déviation, la distribution d'énergie lumineuse du faisceau utile sur la surface d'onde étant globalement conservée. Par faisceau utile il faut comprendre faisceau de traitement laser convergent, l'angle des cônes de focalisation étant sensiblement de l'ordre de 16°, ou toute combinaison de faisceaux permettant soit la visualisation du trajet du faisceau laser de traitement par le médecin ophtalmologue, soit l'éclairage et l'observation de tout point de la chambre antérieure de l'œil à observer. Pour plus de détails sur la constitution du rayonnement utile on pourra se reporter à la demande de brevet précitée au nom de la demanderesse.

Ainsi que représenté sur les figures 1a et 1b, la surface d'onde constituant la face d'entrée 1 est une surface sphérique dont le centre de courbure C est l'image, du point théorique F de l'œil à irradier, formée par la surface réfléchissantes 2. Ainsi que représenté notamment figure 1a, le verre de contact est constitué en un matériau transparent au rayonnement utile sensiblement délimité par une forme présentant un plan de symétrie longitudinal. La forme est constituée sensiblement par une surface cylindrique de révolution 100. Tout mode de réalisation dans lequel la surface cylindrique de révolution 100 est remplacée par une surface cônique admettant un même plan de symétrie longitudinal ne sort pas du cadre de la présente invention. La surface d'entrée sphérique 1 admet pour bord 10 une ligne d'intersection de la surface cylindrique 100 ou de la surface cônique. Dans le cas où la forme du verre est constituée par une surface cylindrique 100, le bord 10 de la surface d'entrée sphérique 1 constituant une ligne d'intersection de la surface cylindrique 100, représente une ellipse. La surface de sortie sphérique 3 admet dans le plan de symétrie longitudinal du verre de contact un axe de révolution Z Z' parallèle à une génératrice 1000 de la surface cylindrique 100. La surface de sortie 3 admet en outre pour bord 30 un cercle de rayon inférieur à celui de la base 20 de la surface cylindrique 100 et non coaxial à ce dernier. Sur la figure 1b la face d'entrée 1 du rayonnement utile est délimitée par le bord 10 de la surface cylindrique 100, le bord 30 de la surface de sortie 3 étant représenté, excentré, en traits mixtes. Le rayonnement utile est constitué par exemple d'un faisceau de traitement 11 dont la trace sur la face d'entrée 1 est représentée, le faisceau de traitement étant accompagné de deux faisceaux tournants de visualisation 110 tangents à l'enveloppe du faisceau 11 et rendant visible, pour le praticien, l'enveloppe du faisceau de traitement 11.

La face d'entrée 1 et la surface de sortie 3 sphériques constituent, pour le verre de contact, respectivement une surface convexe et une surface concave.

L'utilisation du verre de contact par le médecin se faisant par pression du verre sur la cornée élastique, on peut admettre que la surface antérieure de la cornée lors de l'irradiation laser est sphérique et a le même rayon de courbure que la face cornéenne du verre constituée par la surface de sortie 3 du rayonnement du verre de contact. Le rayon de courbure ρ de la surface de sortie sphérique 3 concave est sensiblement égal à 0,8 cm. Cette valeur, supérieure au rayon de courbure de la face antérieure de la cornée d'un œil au repos, permet l'obtention d'un bon contact entre verre et cornée par l'élimination des bulles d'air et offre la possibilité, lors de l'observation de l'angle irido cornéen, d'ouvrir cet angle par pression du verre sur l'œil. Le risque de formation de plis de Decemet peur être évité par mouvement du verre de contact. La surface réfléchissante 2 par réflexion totale est une aire plane délimitée par l'intersection d'un plan et de la surface cylindrique 100 ou de la surface cônique. Ainsi que représenté sur la figure 1b, ce plan forme avec un plan contenant l'axe de révolution Z Z' de la surface de sortie 3 et orthogonal au plan de symétrie longitudinal du verre de contact, un angle dièdre α compris entre 18° et 48° sensiblement. Ce plan contenant l'axe de symétrie Z Z' et orthogonal au plan de symétrie longitudinal du verre de contact est représenté figure 1b par sa trace confondue avec l'axe Z Z'. Sur la figure 1a, sont également représentées des faces 4, intersections de plans et de la surface cylindrique 100 ou cônique. Ces faces 4 permettent par enlèvement de matière à partir du cylindre ou du cône d'alléger le verre de contact. Tout mode de réalisation dans lequel un allègement du verre de contact est obtenu par un moyen différent ne sort pas du cadre de la présente invention. Le matériau utilisé pour la réalisation du verre de contact est de préférence un matériau qui ne présente pas de retrait après contrainte thermique.

Ainsi que représenté figure 1b, la surface réfléchissante 2 par réflexion totale comporte une plaquette de protection 21 ménageant entre la surface réfléchissante 2 et la plaquette 21 une lame d'air 210 assurant la condition de réflexion totale pour le rayonnement utile 11. Cette plaquette permet une protection contre les projections sur la surface réfléchissante 2 de liquides tels le «Méthocel» qui seraient susceptibles de dégrader les conditions de réflexion totale et de densité d'énergie du faisceau laser de traitement. La plaquette 210 est collée, par exemple, avec une résine thermodurcissable. Le centre de courbure C de la face d'entrée sphérique du rayonnement utile 11 est situé dans le plan de symétrie longitudinal du verre de contact à une distance d voisine de 1 cm de l'axe de révolution Z Z' de la surface de sortie 3 du verre de contact.

Cette caractéristique permet une uniformisation des dimensions du verre de contact et permet d'en faciliter la réalisation.

Le verre de contact comporte en outre au niveau du bord 10 de la face d'entrée 1 du rayonnement et au niveau du bord 30 de la surface de sortie 3 un épaulement 101, 300 constitué respectivement par la surface cylindrique libre 100 et par une surface délimitée par l'intersection de la surface cylindrique 100 et d'un cône. Ces épaulements 101, 300 permettent d'entourer le verre de contact d'un étui plastique de protection facilitant la manipulation du verre en gisement $\varphi$ et en rotation $\theta$ autour de l'axe de symétrie Z Z' par le médecin au cours de l'observation et du traitement.

Ainsi pour une valeur déterminée de l'angle dièdre $\alpha$ et pour une plage de valeurs de l'incidence relative du rayonnement utile 11 sur la face d'entrée 1 du verre de contact obtenues par rotation du verre en gisement $\varphi$, le rayonnement utile conservant une direction fixe, et par rotation $\theta$ du verre de contact autour de l'axe Z Z' le point de focalisation F peut, dans l'œil, être déplacé à l'intérieur d'une zone de traitement T sensiblement en forme de couronne circulaire centrée sur l'axe Z Z'.

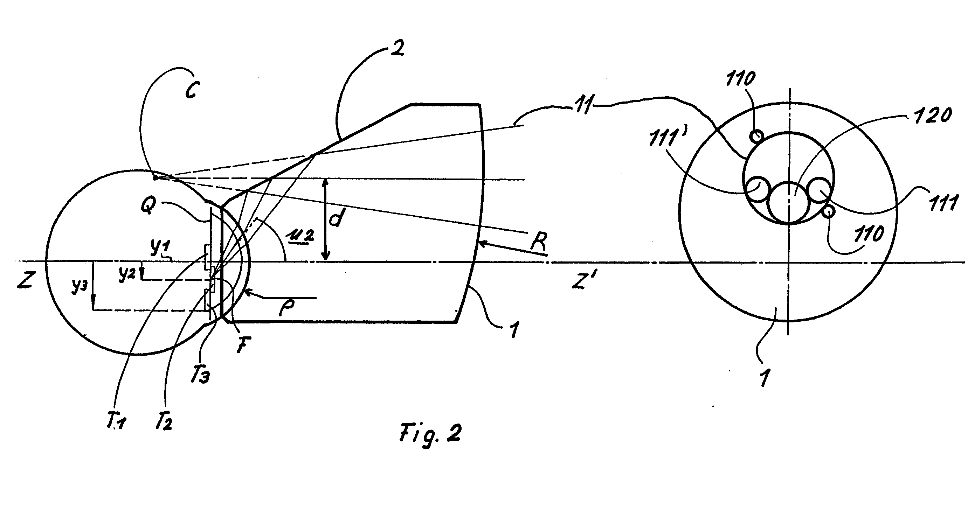

Ainsi que représenté figure 2, la face antérieure de l'iris est assimilée en première approximation au plan tangent Q à la face antérieure du cristallin et perpendiculaire à l'axe Z Z', confondu sur la figure 2 avec l'axe optique de l'œil. Les paramétres géométriques de l'œil sont pour la cornée en présence du verre de contact:

- rayon de courbure $\rho$ de la face antérieure de la cornée     0,8 cm
- épaisseur     0,5 mm
- rayon de courbure de la face postérieure     0,75 cm.

Pour une longueur d'onde d'émission laser $\lambda = 1,06$ μm, laser Nd:YAG, l'indice de la cornée est $n \sim 1,377$, indice de l'humeur acqueuse $n \sim 1,337$, ces valeurs étant obtenues à partir des courbes de Tagawa (Tabulæ biologicæ). Compte tenu des affections diverses à traiter, les tirs laser doivent être effectués dans l'angle irido cornéen, sur l'iris, sur le cristallin et au voisinage du plan Q tangent à la surface antérieure du cristallin situé sensiblement à 3,6 mm de la face antérieure de la cornée. En toute rigueur pour chaque point situé dans ce plan à une distance fixe de l'axe de l'œil, il existe une valeur de $\alpha$, compte tenu du choix du paramètre d distance du centre de courbure C de la face d'entrée à l'axe Z Z' et donc distance de l'axe du faisceau utile à ce même axe, permettant de focaliser le faisceau sur ce point dans des conditions optimales.

Cependant, compte tenu des dispersions des valeurs des paramètres précités en fonction des individus, il est suffisant de définir un nombre déterminé de zones de traitement sur ce plan Q qui, compte tenu de la liberté de manipulation du praticien, peuvent être atteintes chacune sans dégradation notoire des qualités de focalisation au moyen d'un verre de contact de caractéristiques données. Sur la figure 2, trois zones de traitement T1, T2, T3, deux zones voisines se recouvrant partiellement, ont été définies. La ligne médiane de ces zones, circonférence de rayon respectif y1, y2, y3 correspond à une zone moyenne. Le choix des rayons $y1 = 0$, $y2 = 2,23$ mm et $y3 = 5,6$ mm a été effectué en vue de permettre, compte tenu de la distance moyenne d, axe du faisceau utile 11 – axe Z Z', une inclinaison optimale du faisceau utile après réflexion sur la face de réflexion 2. Cette inclinaison u par rapport à l'axe Z Z', pour un cône de focalisation du faisceau de 16° environ, permet notamment d'obtenir une efficacité mécanique maximale de perçage du fait que les faisceaux au voisinage du plan Q sont aussi proches que possible, en accord avec la topographie de la zone à traiter, de la normale à cette zone. Cette efficacité mécanique est de plus conjuguée à un chemin optique maximal du faisceau dans l'œil ce qui a pour conséquence primordiale d'imposer une densité d'énergie minimale du rayonnement au niveau de la face antérieure de la cornée, jonction cornée – verre, et de ce fait d'assurer une sécurité absolue du patient. De plus ces valeurs, pour des tirs sur le cristallin, autorisent des tirs qui cependant évitent l'éclairage des parties très sensibles de la rétine telles que la fovea par exemple. Les valeurs d'inclinaison correspondantes sont:

– $u_1 = 49°$: zone centrale du cristallin. Cette valeur fournit pour le faisceau un chemin optique moyen de 4 mm. Ce trajet relativement court ne permet pas d'effectuer des tirs d'impulsion dont l'énergie est supérieure à 30 à 40 mJ en raison des risques d'instabilité de position du claquage optique.

– $u_2 = 63°$: zone intermédiaire correspondant principalement au traitement de l'iris: cette valeur fournit un chemin optique moyen de 5 à 6 mm et autorise des tirs laser à grande énergie 50 à 100 mJ.

– $u_3 = 71°$: zone de l'angle irido cornéen, cette valeur fournit un chemin optique moyen de 9 à 10 mm et autorise des tirs à très haute énergie 100 à 150 mJ.

L'ensemble des différents paramètres précédents permet de définir un verre de contact particulier, par zone de traitement, permettant ainsi préférentiellement le traitement de la zone correspondante. Ainsi pour chaque zone T1, T2, T3 et pour les paramètres correspondants $y_1$, $u_1$; $y_2$, $u_2$;

$y_3$, $u_3$ l'angle dièdre $\alpha$ constitué par le plan délimitant la surface réfléchissante par réflexion totale et le plan orthogonal au plan de symétrie longitudinal du verre et contenant l'axe de symétrie Z Z' de la surface de sortie 3 du rayonnement utile est choisi parmi les valeurs $\alpha_1 = 23°$, $\alpha_2 = 28°$, $\alpha_3 = 32°$, correspondant respectivement aux paramètres $y_1$, $u_1$; $y_2$, $u_2$; $y_3$, $u_3$ précédemment définis.

La face d'entrée de tout verre de contact ainsi obtenue comporte de préférence un rayon de courbure R sensiblement égal à 4 cm. Ainsi, comme représenté sur la figure 2, l'image d'un faisceau utile constitué, lors de l'observation, par des faisceaux de visualisation tournants 110 du faisceau de traitement 11 utilisé ultérieurement par le praticien, par les faisceaux d'observation 111 et 111' et par un faisceau d'éclairage 120 est exempte de recouvrement entre faisceau d'observation et faisceau d'éclairage évitant ainsi l'éblouissement du praticien par réflexion du faisceau d'éclairage sur la face d'entrée 1. Pour plus de détails sur la constitution du faisceau utile on peut se reporter à la demande de brevet au nom de la demanderesse déjà citée.

L'ensemble des trois verres de contact ainsi définis constitue un jeu permettant au praticien l'observation ou l'irradiation optimales de la chambre antérieure de l'œil. L'angle dièdre $\alpha$ constitué par le plan délimitant la surface réfléchissante par réflexion totale et le plan perpendiculaire au plan de symétrie longitudinal du verre et contenant l'axe de révolution Z Z' de la surface de sortie 3 du rayonnement utile a, pour chacun des verres du jeu, une valeur différente égale à l'une des trois valeurs remarquables. Ce jeu permet d'assurer l'irradiation ou l'observation de l'ensemble des points de la chambre antérieure de l'œil.

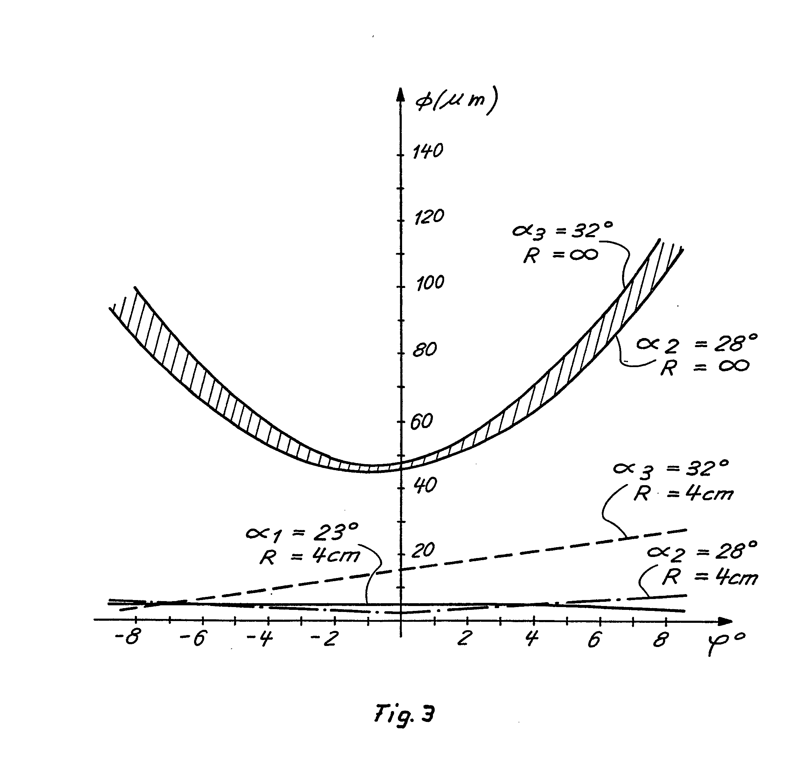

Conformément à la figure 3, les courbes représentent l'aberration mesurée en micromètres et définie comme l'accroissement de diamètre d'un spot de focalisation de 60 μm pour un faisceau dont le cône de focalisation est sensiblement 16°, en fonction de l'angle d'incidence du faisceau utile 11 sur la face réfléchissante 2, le verre étant soumis à une rotation en gisement $\varphi$, ceci pour les trois verres de contact constituant le jeu selon l'invention et pour un verre du type verre de Goldmann pour lequel le rayon de courbure de la face d'entrée est infini, $R = \infty$. La zone hachurée correspondant à la disperson de l'aberration pour une face d'entrée plane du type verre de Godmann en fonction de l'angle dièdre $\alpha$ du plan constituant la surface réfléchissante et du plan contenant l'axe Z Z' orthogonal au plan de symétrie longitudinal du verre de contact, il est aisé de voire que l'aberration maximale apportée par un verre de contact selon l'invention, très faible par rapport au diamètre du spot de focalisation, est environ trois fois moins importante, dans le cas le plus défavorable, c'est à dire celui du verre adapté au traitement de l'angle irido-cornéen pour lequel $\alpha_3 - 32°$, que dans le cas d'un verre du type Goldmann.

On a ainsi décrit un système de verres de contact pour l'observation ou l'irradiation laser pour lesquels un grand facteur de sécurité est atteint pour le patient. A cette qualité doit encore s'ajouter celle d'un grand confort d'utilisation pour le praticien. En effet, le faisceau utile étant sensiblement parallèle à l'axe optique de l'œil, l'observation de toutes les parties à traiter de la chambre antérieure de l'œil est obtenue sans modification notoire de la position patient – médicin par seule manipulation du verre de contact. Cette manipulation peur être effectuée par rotation $\theta$ de 360° autour de l'axe Z Z' de symétrie de la face de sortie 3 du verre et par rotation en gisement $\varphi$ du verre de contact. La seule limitation à envisager pour l'amplitude des rotations en gisement $\varphi$ est celle de la perte de la condition de réflexion totale sur la face réfléchissante 2. Pour chacun des trois verres définis précédemment, l'amplitude de rotation maximale du verre dans le sens critique, c'est-à-dire le sens tendant à fermer l'angle d'incidence du faisceau utile 11 sur la surface réfléchissante 2, est

$\varphi_1 = 17, 43°$      pour $\alpha_1 = 23°$
$\varphi_2 = 12, 43°$      pour $\alpha_2 = 28°$
$\varphi_3 = 8, 43°$      pour $\alpha_3 = 32°$

pour un verre d'indice 1,507 à $\lambda = 1.06$ μ.

Ces valeurs limites de rotation en gisement par rapport à une position initiale de départ correspondent sensiblement à la coïncidence le l'axe optique de l'œil et de l'axe de symétrie Z Z' de la surface de sortie 3. Elles sont largement suffisantes pour permettre au médecin de compenser, par seule manipulation du verre de contact, les mouvements erratiques du globe oculaire du patient existant malgré une anesthésie préalable des muscles du globe oculaire, ou pour atteindre, sans dégradation sensible des conditions de focalisation du faisceau, donc avec une assurance de fiabilité et de reproductibilité des opérations, chaque point des zones de traitement correspondantes, respectivement.

**Revendications**

1. Verre de contact pour l'observation ou l'irradiation de l'œil compremant une face d'entrée (1), une surface plane réfléchissante par réflexion totale (2) et une surface de sortie sensiblement sphérique (3), caractérisé en ce que ladite face d'entrée (1) constitue une surface d'onde pour le rayonnement convergent utilisé pour l'irradiation, ledit rayonnement ne subissant ainsi aucune déviation en entrant dans le verre.

2. Verre de contact selon la revendication 1, caractérisé en ce que ladite face d'entrée (1) est une surface sphérique dont le centre de courbure (C) est l'image du point théorique (F) de l'œil à observer ou irradier, formée par ladite surface réfléchissante (2).

3. Verre de contact selon la revendication 1 ou 2, caractérisé en ce qu'il est constitué en un matériau transparent sensiblement délimité par une forme présentant un plan de symétrie longitudinal et constituée par une surface cylindrique de révolution (100), ladite face d'entrée (1) admettant pour bord (10) son intersection avec la surface cylindrique (100) et la surface de sortie sphérique (3) ayant un axe de révolution (Z Z') contenu dans le plan de symétrie longitudinal du verre et parallèle à une génératrice de la surface cylindrique (100), ladite surface de sortie (3) admettant en outre pour bord (30) un cercle de rayon inférieur à celui de la base (20) de la surface cylindrique (100) et non coaxial à ce denier, ladite face d'entrée (1) et la surface de sortie (3) sphériques constituant respectivement une surface convexe et une surface concave du verre de contact, ladite surface plane réfléchissante par réflexion totale (2) étant un plan délimité par son intersection avec la surface cylindrique (100), ledit plan constituant avec un plan contenant l'axe de révolution (Z Z') de la surface de sortie (3) et orthogonal au plan de symétrie longitudinal du verre de contact un angle dièdre (α) compris sensiblement entre 18° et 48°.

4. Verre de contact selon la revendication 3, caractérisé en ce que ladite surface réfléchissante par réflexion totale (2) comporte une plaquette de protection (21) ménageant entre la surface réfléchissante et la plaquette une lame d'air (210) assurant la condition de réflexion totale pour le rayonnement utilisé pour l'irradiation.

5. Verre de contact selon l'une des revendications 3 ou 4, caractérisé en ce que la surface de sortie sphérique concave (3) a un rayon de courbure sensiblement égal à 0,8 cm.

6. Verre de contact selon l'une des revendications 3 à 5, caractérisé en ce que le centre de courbure (C) de la face d'entrée sphérique (1) est situé, dans le plan de symétrie longitudinal du verre de contact, à une distance (d) sensiblement égale à 1 cm de l'axe de révolution (Z Z') de la surface de sortie (3) du verre de contact.

7. Verre de contact selon l'une des revendications 2 à 6, caractérisé en ce que le rayon de courbure de la surface sphérique d'entrée est sensiblement égal à 4 cm.

8. Verre de contact selon l'une des revendications 3 à 7, caractérisé en ce que le verre de contact comporte au niveau du bord (10) de la face d'entrée (1) et au niveau du bord (30) de la surface de sortie (3) un épaulement constitué respectivement par la surface cylindrique (100) libre et par une surface cônique délimitée par son intersection avec la surface cylindrique (100) et avec le plan de la face réfléchissante (2).

9. Verre de contact selon l'une des revendications 3 à 8, caractérisé en ce que la valeur de l'angle dièdre (α) constitué par le plan de la surface réfléchissante par réflexion totale (2) et le plan orthogonal au plan de symétrie longitudianl du verre contenant l'axe de révolution (Z Z') de la surface de sortie (3) est choisie parmi les trois valeurs 32°, 28° et 23°.

10. Jeu de verres de contact pour l'observation ou l'irradiation de l'œil, caractérisé en ce qu'il comprend trois verres de contact, selon la revendication 9, chacun des-dits verres de contact ayant un angle dièdre (α) différent de celui des deux autres verres, ledit jeu permettant d'assurer l'irradiation de l'ensemble des points de la chambre antérieure de l'œil.

**Patentansprüche**

1. Kontaktglas zur Beobachtung oder Bestrahlung des Auges, umfassend eine Eintrittsfläche (1), eine plane durch Totalreflexion reflektierende Fläche und eine im wesentlichen sphärische Austrittsfläche (3), dadurch gekennzeichnet, dass die besagte Eintrittsfläche (1) eine Wellenfläche für die konvergente Strahlung, die zur Bestrahlung verwendet wird, bildet, so dass die besagte Strahlung keiner Ablenkung beim Eintreten in das Glas unterliegt.

2. Kontaktglas gemäss Anspruch 1, dadurch gekennzeichnet, dass die besagte Eintrittsfläche (1) eine sphärische Fläche ist, deren Krümmungsmittelpunkt (C) das Bild des theoretischen Punktes (F) des zu betrachtenden oder zu bestrahlenden Auges ist, das durch die besagte reflektierende Fläche (2) gebildet wird.

3. Kontaktglas gemäss Anspruch 1 oder 2, dadurch gekennzeichnet, dass es aus einem transparenten Material im wesentlichen begrenzt durch eine Form, gebildet ist, die eine longitudinale Symmetrieebene aufweist und durch eine zylindrische Drehfläche (100) gebildet wird, wobei die besagte Eintrittsfläche (1) als Rand (10) ihren Schnitt mit der zylindrischen Fläche (100) zulässt und die sphärische Austrittsfläche (3) eine Drehachse (Z Z') besitzt, die in der longitudinalen Symmetrieebene des Glases enthalten und parallel zu einer Erzeugenden der zylindrischen Fläche (100) ist, wobei die besagte Austrittsfläche (3) zudem als Rand (30) einen Kreis mit einem Radius kleiner als derjenige der Basis (20) der zylindrischen Fläche (100) zulässt und nicht koaxial zu dieser letzteren ist, wobei die besagte Eintrittsfläche (1) und die Austrittsfläche (3), die sphärisch sind, eine konvexe Fläche bzw. eine konkave Fläche des Kontaktglases bilden, wobei die besagte plane durch Totalreflexion reflekttierende Fläche (2) eine Ebene ist, die durch ihren Schnitt mit der zylindrischen Fläche (100) begrenzt ist, wobei die besagte Ebene mit einer Ebene, die die Drehachse (Z Z') der Austrittsfläche (3) enthält, einen Flächenwinkel (α) bildet, der im wesentlichen zwischen 18° und 48° liegt.

4. Kontaktglas nach Anspruch 3, dadurch gekennzeichnet, dass die besagte durch Totalreflexion reflektierende Fläche (2) eine Schutzplatte (21) aufweist, wobei zwischen der reflektierenden Fläche und der Platte ein Luftspalt (210) angeordnet ist, der den Zustand der Totalreflektion für die zur Bestrahlung verwendete Strahlung sicherstellt.

5. Kontaktglas gemäss einem der Ansprüche 3 oder 4, dadurch gekennzeichnet, dass die konkave sphärische Austrittsfläche (3) einen Krümmungsradius im wesentlichen gleich 0,8 cm besitzt.

6. Kontaktglas gemäss einem der Ansprüche 3 bis 5, dadurch gekennzeichnet, dass das Krümmungszentrum (C) der sphärischen Eintrittsfläche (1) in der longitudinalen Symmetrieebene des Kontaktglases in einem Abstand (d) im wesentlichen gleich 1 cm von der Drehachse (Z Z') der Austrittsfläche (3) des Kontaktglases angeordnet ist.

7. Kontaktglas gemäss einem der Ansprüche 2 bis 6, dadurch gekennzeichnet, dass der Krümmungsradius der sphärischen Eintrittsfläche im wesentlichen gleich 4 cm ist.

8. Kontaktglas gemäss einem der Ansprüche 3 bis 7, dadurch gekennzeichnet, dass das Kontaktglas in Höhe des Randes (10) der Eintrittsfläche (1) und in Höhe des Randes (30) der Austrittsfläche (3) eine Schulter aufweist, die durch die freie zylindrische Fläche (100) bzw. durch eine konische Fläche gebildet wird, die durch ihren Schnitt mit der zylindrischen Fläche (100) und mit der Ebene der reflektierenden Fläche (2) begrenzt ist.

9. Kontaktglas gemäss einem der Ansprüche 3 bis 8, dadurch gekennzeichnet, dass die Grösse des Flächenwinkels ($\alpha$), der durch die Ebene der durch Totalreflexion reflektierenden Fläche (2) und die Ebene orthogonal zur longitudinalen Symmetrieebene gebildet wird, die die Drehachse (Z Z') der Austrittsfläche (3) enthält, aus den drei Werten 32°, 28° und 23° ausgewählt ist.

10. Kontaktgläserersatz zur Beobachtung oder Bestrahlung des Auges, dadurch gekennzeichnet, dass er drei Kontaktgläser gemäss Anspruch 9 aufweist, wobei jedes der besagten Kontaktgläser einen Flächenwinkel ($\alpha$) aufweist, der verschieden zu demjenigen der beiden anderen Gläser ist, wobei der besagte Satz ermöglicht, die Bestrahlung der Gesamtheit von Stellen der vorderen Augenkammer sicherzustellen.

**Claims**

1. Contact lens for observing or irradiating the eye comprising an entry face (1), a totally reflecting planar surface (2) and an essentially spherical exit surface (3) characterized in that said entry face (1) constitutes a wave surface for the convergent rays employed for irradiation, said rays thereby undergoing no deviation on entering the lens.

2. Contact lens according to claim 1 characterized in that said entry face (1) is a spherical surface the center of curvature (C) of which is the image of the theoretical point (F) of the eye to be observed or irradiated formed by said reflecting surface (2).

3. Contact lens according to claims 1 or 2 characterized in that it is realized in transparent material essentially bounded by a form having a longitudinal plane of symmetry and constituted by a cylindrical surface of revolution (100), said entry face (1) admitting as limit (10) its intersection with the cylindrical surface (100) and the spherical exit surface (3) having an axis of revolution (Z Z') contained in the longitudinal plane of symmetry of the lens and parallel to a generatrix of the cylindrical surface (100), said exit surface additionally admitting as limit (30) a circle of radius less than that of the base (20) of the cylindrical surface (100) and non-coaxial with this latter, said spherical entry face (1) and exit surface (3) constituting respectively a convex and concave surface of the contact lens, said totally reflecting planar surface (2) being a plane bounded by its intersection with the cylindrical surface (100) and forming a dihedral angle ($\alpha$) substantially between 18° and 48° with a plane containing the axis of revolution (Z Z') of the exit surface (3) and orthogonal to the longitudinal plane of symmetry of the contact lens.

4. Contact lens according to claim 3 characterized in that said totally reflecting surface (2) comprises a protection plate (21) arranged to provide a layer of air (210) between the reflecting surface and the plate thereby to assure total reflection of the rays employed for irradiation.

5. Contact lens according to either of claims 3 or 4 characterized in that the concave spherical exit surface (3) has a radius of curvature substantially equal to 0.8 cm.

6. Contact lens according to any of claims 3–5 characterized in that the center of curvature (C) of the spherical entry face (1) is located in the longitudinal plane of symmetry of the contact lens at a distance (d) substantially equal to 1 cm. from the axis of revolution (Z Z') of the exit surface of the contact lens.

7. Contact lens according to any of claims 2–6 characterized in that the radius of curvature of the spherical entry surface is substantially equal to 4 cm.

8. Contact lens according to any of claims 3–7 characterized in that the contact lens comprises proximate the limit (10) of the entry face (1) and proximate the limit (30) of the exit surface (3) a shoulder formed respectively by the free cylindrical surface (100) and by a conical surface bounded by its intersection with the cylindrical surface (100) and with the plane of the reflecting surface. (2).

9. Contact lens according to any of claims 3–8 characterized in that the value of the dihedral angle ($\alpha$) formed by the plane of the totally reflecting surface (2) and the plane orthogonal to the longitudinal plane of symmetry of the lens containing the axis of revolution (Z Z') of the exit surface (3) is chosen from among the three values 32°, 28° and 23°.

10. A set of contact lenses for observing or irradiating the eye characterized in that it comprises three contact lenses in accordance with claim 9, each of said contact lenses having a dihedral angle different from that of the two other lenses, said set enabling to assure the irradiation of the totality of points within the frontal chamber of the eye.

Fig. 1a

Fig. 1b

Fig. 2

Tig. 3